(19) Europäisches Patentamt
European Patent Office
Office européen des brevets

(11) Veröffentlichungsnummer: **0 124 723**
**B1**

(12) # EUROPÄISCHE PATENTSCHRIFT

(45) Veröffentlichungstag der Patentschrift: **07.01.87**

(51) Int. Cl.⁴: **C 07 C 69/757**, C 07 C 67/317, C 07 C 67/333

(21) Anmeldenummer: **84102727.9**

(22) Anmeldetag: **13.03.84**

(54) **Verfahren zur Einstellung des Gehalts an trans-Isomeren des Caronaldehyddiacetals.**

(30) Priorität: **08.04.83 DE 3312608**

(43) Veröffentlichungstag der Anmeldung:
**14.11.84 Patentblatt 84/46**

(45) Bekanntmachung des Hinweises auf die Patenterteilung:
**07.01.87 Patentblatt 87/2**

(84) Benannte Vertragsstaaten:
**DE FR GB NL**

(56) Entgegenhaltungen:
**EP - A - 0 031 932**
**EP - A - 0 059 659**
**GB - A - 2 084 151**

(73) Patentinhaber: **DYNAMIT NOBEL AKTIENGESELLSCHAFT, Postfach 1209, D-5210 Troisdorf, Bez. Köln (DE)**

(72) Erfinder: **Schmidt, Hans-Georg, Dr., Porzer Strasse 15, D-5216 Niederkassel (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Gegenstand der vorliegenden Erfindung ist ein Verfahren zur Einstellung eines gewünschten Gehaltes an trans-Isomeren des Cyclopropancarbonsäureesters der Formel

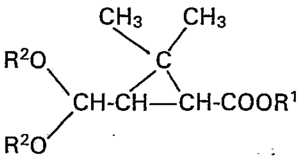

in der $R^1$ und $R^2$ Alkylreste mit 1 bis 4 C-Atomen sind, bei oder nach der Cyclisierung von Pentansäureestern der Formel

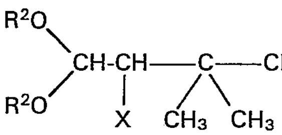

in der $R^1$ und $R^2$ die oben genannte Bedeutung haben und X für Chlor oder Brom steht.

Bei dieser Reaktion, die in Gegenwart von Basen abläuft, entsteht ein Gemisch aus cis-/trans-Verbindungen mit unterschiedlichen Gehalten an den Isomeren. Diese Produkte werden in bekannter Weise zur Herstellung von Pyrethroiden eingesetzt. Die insektizide Wirkung der Pyrethoride hängt deutlich von den sterischen Faktoren des Cyclopropanrings ab; so ist z.B. das Wirkungspotential von cis- und trans-Isomeren (bezüglich des Cyclopropanrings) sehr unterschiedlich (vgl. M. Elliot «Synthetic Pyrethroids», ACS Symposium Series 42, American Chemical, Society, 1977). Daraus ergibt sich, dass die für die Herstellung der Pyrethroide eingesetzten Cyclopropancarbonsäureverbindungen bereits bestimmte Gehalte an cis/trans-Isomeren enthalten sollen, um optimale Wirkungen der daraus hergestellten Pyrethroide zu erreichen.

Es bestand deshalb die Aufgabe, ein Verfahren zu finden, den Gehalt an einem der beiden Isomeren auf bestimmte, reproduzierbare Werte einzustellen.

In Erfüllung dieser Aufgabe wurde nun ein Verfahren zum Einstellen des Gehalts an trans-Isomeren des Cyclopropancarbonsäureesters der Formel I

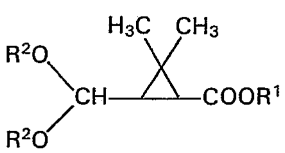
(I)

in der $R^1$ und $R^2$ für Alkylreste mit 1 bis 4 C-Atomen steht, bei oder nach der Cyclisierung von Pentansäureestern der Formel II

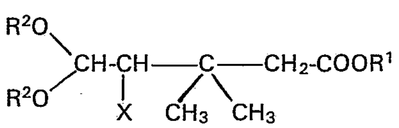
(II)

in der $R^1$ und $R^2$ die oben genannte Bedeutung haben, und X für Chlor oder Brom steht, in Gegenwart von Basen gefunden, das dadurch gekennzeichnet ist, dass man die im Anspruch 1 genannten Massnahmen durchführt.

Der Erfindung liegt die neue Erkenntnis zugrunde, dass die Isomerisierung von 2,2-Dimethyl-3-formyl-(dialkylacetal)-cyclopropancarbonsäurealkylestern, die im folgenden auch als Caronaldehydacetale bezeichnet werden, einerseits durch bestimmte Lösungsmittel verzögert und andererseits durch andere Lösungsmittel gefördert wird und dass bei einer thermischen Belastung eines Isomerengemischs von Caronaldehydacetalen in Gegenwart von Basen sich das cis-trans-Isomerenverhältnis weitgehend zugunsten des trans-Isomeren verschiebt.

Lösungsmittel, die die Bildung der trans-Isomeren nicht fördern, sind solche mit hohen Dielektrizitätskonstanten (über 20), wie zum Beispiel Nitrile oder Alkohole, hauptsächlich Alkylnitrile und Alkylalkohole, deren Alkylgruppe 1 bis 4 C-Atome besitzt. Wenn die Cyclisierung der obengenannten Pentancarbonsäurederivate in Gegenwart dieser Lösungsmittel bei 50°C durchgeführt wird, enthält das gebildete Isomerengemisch des Caronaldehydacetals nur zwischen 45 und 55% des trans-Isomeren. Die Menge des Lösungsmittels soll dabei, wenn dieser relativ hohe Anteil des cis-Isomeren gewünscht wird, mindestens 5 Mol pro Mol des Pentansäurederivats betragen. Bei einem geringen Lösungsmitteleinsatz tritt teilweise Isomerisierung des cis-Isomeren zum trans-Isomeren auf.

Eine teilweise Isomerisierung zum trans-Isomeren tritt bei Anwendung dieser Lösungsmittel auch dann auf, wenn die Cyclisierungstemperatur zu hoch oder die Dauer der Cyclisierungsreaktion zu lange gewählt wird. Der Grad dieser Isomerisierung hängt selbstverständlich von der Temperatur, der Menge und Art des Lösungsmittels sowie der Zeit ab. Bevorzugt wird deshalb die Cyclisierung bei Temperaturen zwischen 30 und 60°C durchgeführt, wenn die genannten hohen Gehalte an dem cis-Isomeren des Caronaldehydacetals gewünscht werden. Kurzfristig kann jedoch auch bei Temperaturen bis zu 120°C die Cyclisierung durchgeführt werden.

Wenn das Isomerengemisch einen Anteil von mehr als etwa 80% des trans-Isomeren enthalten soll, dann wird die oben genannte Cyclisierungsreaktion vorzugsweise in Lösungsmitteln mit Dielektrizitätskonstanten unterhalb von 15, wie Ethern, Trialkylaminen, Orthoameisensäureestern und aliphatischen oder aromatischen Kohlenwasserstoffen durchgeführt. Bevorzugte Ether sind solche mit Siedepunkten über 30, vorzugsweise über 50°C, die ggf. auch mehrere Ethergruppierungen enthalten können, wie z.B. die Methyl- oder Ethylether des Di- oder Triglykols. Auch cyclische Ether, wie z.B. Tetrahydrofuran, sind geeignet.

Beispiele für besonders geeignete Trialkylamine sind Triethylaminhydrochlorid oder Tri-n-butylamin. An einsetzbaren Orthoameisensäureestern seien die Methyl- und Ethylester der Orthoameisensäure genannt.

Kohlenwasserstoffe, die die Bildung des trans-Isomeren beschleunigen, sind aliphatische, unsubstituierte gesättigte Kohlenwasserstoffe mit 5 bis 12 C-Atomen, Benzol, die Toluole und Xylole.

Diese Lösungsmittel, die die Cyclisierung in Richtung des trans-Isomeren begünstigen, können in beliebiger Menge bei der Cyclisierungsreaktion eingesetzt werden. Die bevorzugte Lösungsmittelmenge liegt auch hier bei mehr als 5 Mol pro Mol des Pentancarbonsäureesters.

Beim Einsatz der genannten Lösungsmittel, die die Bildung der trans-Isomeren des Caronaldehydacetals begünstigen, kann die Cyclisierungsreaktion im Temperaturbereich zwischen 0 und 150°C durchgeführt werden.

Wie breits oben gesagt, wird das Isomerengleichgewicht der Caronaldehydacetale auch dann in Richtung des trans-Isomeren begünstigt, wenn das Isomerengemisch in Gegenwart von Basen erhitzt wird. Die Menge der Base braucht dabei nur in katalytischer Grössenordnung zu liegen; es genügen 0,01 bis 5 Gew.-%, bezogen auf das Caronaldehydacetal. Als Base wird bevorzugt ein Alkalialkoholat eingesetzt, wobei die Estergruppierung nach Möglichkeit die gleiche wie die Ester- oder Acetalgruppierung des Caronaldehydacetals sein soll.

Die Isomerisierung des cis-Isomeren zum trans-Isomeren in Gegenwart von Basen kann ebenfalls ab Temperaturen von 40°C durchgeführt werden. Bei tiefen Temperaturen stellt sich jedoch nur sehr langsam das Gleichgewicht ein, während es sich bei höheren Temperaturen, z.B. ab 100°C schneller einstellt. Es ist deshalb auch möglich, bei höheren Temperaturen die thermische Isomerisierung auch in Gegenwart von Alkoholen oder Nitrilen als Lösungsmittel durchzuführen, wenn die Temperatureinwirkung genügend lange erfolgt.

Das erfindungsgemässe Verfahren ermöglicht es, praktisch jeden Gehalt zwischen ca. 45 und 95% an trans-Isomeren in dem Caronaldehydacetalgemisch einzustellen. Wenn ein relativ geringer Gehalt an trans-Isomeren gewünscht wird, dann wird die Cyclisierungsreaktion in Gegenwart von Alkoholen oder Nitrilen bei möglichst niedriger Temperatur durchgeführt. In dem dabei erhaltenen Gemisch lässt sich dann durch Erhitzen im alkalischen Medium der trans-Gehalt auf jeden gewünschten höheren Gehalt erhöhen.

Erfindungsgemäss ist es auch möglich, bereits bei der Cyclisierungsreaktion einen gewünschten bestimmten Anteil an trans-Isomeren in dem oben genannten Bereich zu erhalten, wenn man ein Gemisch der genannten Lösungsmittel einsetzt, wobei durch Vorversuche bestimmt wurde, welcher Bereich an trans-Isomeren beim Einsatz eines gewählten Gemischs erhalten wird.

Die als Ausgangsprodukte für die Cyclisierungsreaktion einzusetzenden Patentsäurederivate werden nach an sich bekannten Verfahren, wie z.B. in der DE-OS 3 046 059 beschrieben sind, hergestellt. Auch die Cyclisierung erfolgt in an sich bekannter Weise in Gegenwart von Alkalialkoholaten, deren Alkoholkomponente bis zu 8 C-Atomen betragen kann. Dabei werden die Alkoholate mindestens im stöchiometrischen Verhältnis zu dem Pentansäurederivat eingesetzt. Das bevorzugte Molverhältnis Pentansäurederivat : Alkalialkoholat liegt zwischen 1 : 1 und 1 : 1,5.

Die folgenden Beispiele erläutern die Erfindung.

*Beispiel 1*

Eine Mischung aus 28,3 g 3,3-Dimethyl-4-brom-5,5-dimethoxypentansäureethylester, 115 g Tetrahydrofuran und 5,9 g $NaOCH_3$ wird während drei Stunden unter Rühren und Feuchtigkeitsausschluss auf 50°C erhitzt. Das entstehende Salz wird nach dem Abkühlen abfiltriert, das Lösungsmittel des Filtrats im Vakuum entfernt und der Filtratrückstand im Vakuum destilliert. Man erhält 17,2 g eines cis-trans-Gemisches des 2,2-Dimethyl-3-formyl(dimethylacetal)-cyclopropancarbonsäuremethylesters. Das Isomeren-Verhältnis beträgt cis : trans = 7 : 93.

*Beispiel 2*

Analog zu Beispiel 1 werden 24 g 3,3-Dimethyl-4-chlor-5,5-dimethoxy-pentansäuremethylester umgesetzt. Die Reaktionstemperatur beträgt 65°C, die Reaktionsdauer fünf Stunden. Man erhält 17,2 g 2,2-Dimethyl-3-formyl(dimethylacetal)-cyclopropancarbonsäuremethylester. Isomerenverhältnis cis : trans = 18 : 82.

*Beispiel 3*

Analog zu Beispiel 1 werden 32 g 3,3-Dimethyl-4-brom-5,5-diethoxy-pentansäureethylester mit 8,4 g $NaOC_2H_5$ in 115 g Tetrahydrofuran umgesetzt. Reaktionszeit 30 Min., Ausbeute: 18,9 g, Isomerenverhältnis cis : trans = 3 : 97.

*Beispiel 4*

28,3 g 3,3-Dimethyl-4-brom-5,5-dimethoxypentansäuremethylester werden in 147 g Toluol mit 6,7 g $NaOCH_3$ analog Beispiel 1 zu 16,2 g Cyclopropanverbindung umgesetzt. Isomerenverhältnis cis : trans = 10 : 90.

*Beispiel 5*

Es wurde analog Beispiel 1 gearbeitet, jedoch das $NaOCH_3$ durch 13,9 g K-tert.Butylat ersetzt. Es wurden 18,1 g der Cyclopropanverbindung erhalten (Reaktionszeit 1,5 Stunden). Isomerenverhältnis cis : trans = 6 : 94.

*Beispiel 6*

28,3 g 3,3-Dimethyl-4-brom-5,5-dimethoxypentansäuremethylester werden analog zu Beispiel 1 in 57 g Methanol mit 6,7 $NaOCH_3$ umgesetzt (Reaktionsdauer 13 Stunden). Man erhält 17,6 g der Cyclopropanverbindung. Isomerenverhältnis cis : trans = 49 : 51.

*Beispiel 7*

9,5 g 3,3-Dimethyl-4-chlor-5,5-dimethoxypentansäuremethylester werden mit 2,7 g $NaOCH_3$ in 29,5 g Acetonitril bei 50°C innerhalb von drei Stunden umgesetzt. Ausbeute an Cyclopropanverbindung 6,3 g. Isomerenverhältnis cis : trans = 50 : 50.

*Beispiel 8*

7,4 g 2,2-Dimethyl-3-formyl(dimethylacetal)-cyclopropancarbonsäuremethylester (cis : trans-Verhältnis = 50 : 50) werden mit 0,07 g $NaOCH_3$ fünf Stunden auf 100°C erhitzt. Anschliessend wird das Produkt im Vakuum abdestilliert. Man erhält 6,8 g

2,2-dimethyl-3-formyl(dimethylacetal)-cyclopropancarbonsäuremethylester mit dem Isomerenverhältnis cis : trans = 6 : 94.

**Patentansprüche**

1. Verfahren zur Einstellung eines bestimmten Gehaltes an trans-Isomeren des Cyclopropancarbonsäureesters der Formel

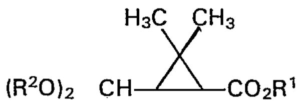

(I)

in der $R^1$ und $R^2$ Alkylreste mit 1 bis 4 C-Atomen bedeuten bei oder nach der Cyclisierung von Pentansäurederivaten der Formel

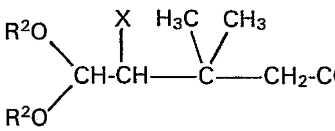

(II)

in der $R^1$ und $R^2$ die oben genannte Bedeutung haben und X für Chlor oder Brom steht, dadurch gekennzeichnet, dass man

A) zur Einstellung eines Gehalts zwischen 45 und 55 Gew.-% des trans-Isomeren die Cyclisierung in Gegenwart von Alkoholen oder Alkylnitrilen als Lösungsmittel durchführt,

oder

B) zur Einstellung eines Gehalts über 80% an trans-Isomeren entweder

a) die Cyclisierung in Gegenwart von Lösungsmitteln aus der Gruppe der Ether, Trialkylamine, Orthoameisensäureester oder Kohlenwasserstoffe durchführt, oder

b) einen Cyclopropancarbonsäureester der Formel I in Gegenwart von 0,01 bis 5 Gew.-% einer Base erhitzt.

2. Verfahren gemäss Anspruch 1, dadurch gekennzeichnet, dass man zur Einstellung eines Gehalts zwischen 45 und 55 Gew,-% des trans-Isomeren die Cyclisierung bei Temperaturen zwischen 30 und 60°C durchführt.

3. Verfahren gemäss Anspruch 1, dadurch gekennzeichnet, dass man zur Einstellung eines Gehalts über 80% an trans-Isomeren den Cyclocarbonsäureester der Formel I in Gegenwart von 0,01 bis 5 Gew.-% einer Base auf Temperaturen über 100°C erhitzt.

**Claims**

1. Process for adjusting a specified trans-isomer content of the cyclopropanecarboxylic acid ester of the formula

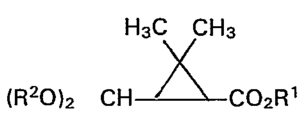

(I)

in which $R^1$ and $R^2$ denote alkyl residues with 1 to 4 C-atoms in or after the cyclising of pentanoic acid derivatives of the formula

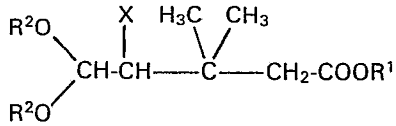

(II)

in which $R^1$ and $R^2$ have the above-indicated meaning and X stands for chlorine or bromine, characterised in that

A) for adjusting to a content between 45 and 55% by weight of the trans-isomers, the cyclisation is carried out in the presence of alcohols or alkyl nitriles as solvents,

or

B) for adjusting to a content above 80% of trans-isomers either

a) the cyclising is carried out in the presence of solvents from the group of ethers, trialkylamines, orthoformic acid esters or hydrocarbons, or

b) a cyclopropanecarboxylic acid ester of formula I is heated in the presence of 0.01 to 5% by weight of a base.

2. Process according to claim 1, characterised in that, for adjusting to a trans-isomer content between 45 and 55% by weight, the cyclising is carried out at temperatures between 30 and 60°C.

3. Process according to claim 1, characterised in that, for adjusting to a trans-isomer content above 80%, the cyclocarboxylic acid ester of formula I is heated in the presence of 0.01 to 5% by weight of a base to temperatures above 100°C.

**Revendications**

1. Procédé pour régler à une valeur déterminée la teneur en isomère trans de l'ester d'acide cyclopropanecarboxylique de formule:

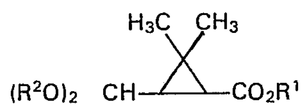

(I)

(dans laquelle $R^1$ et $R^2$ représentent des restes alkyles ayant 1 à 4 atomes de carbone) pendant ou après la cyclisation de dérivés d'acides pentanoiques de formule:

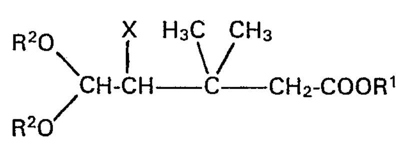

(II)

(dans laquelle $R^1$ et $R^2$ ont le sens précité et X représente un atome de chlore ou de brome), procédé caractérisé en ce que:

A) pour régler la teneur en isomère trans à une valeur comprise entre 45 et 55% en poids, on conduit la cyclisation en présence d'alcools ou d'alkylonitriles comme solvants,

ou

B) pour régler la teneur en isomère trans à une valeur supérieure à 80%,

a) on conduit la cyclisation en présence de solvants du groupe des éthers, des trialkylamines, des esters de l'acide orthoformique ou des hydrocarbures, ou bien

b) on chauffe un ester d'acide cyclopropanecarboxylique de formule I en présence de 0,01 à 5% en poids d'une base.

2. Procédé selon la revendication 1, caractérisé en ce que, pour régler la teneur en isomère trans à une valeur comprise entre 45 et 55%, on conduit la cylisation à une température comprise entre 30 et 60°C.

3. Procédé selon la revendication 1, caractérisé en ce que, pour régler la teneur en isomère trans à une valeur supérieure à 80%, on chauffe l'ester d'acide cyclocarboxylique de formule I en présence de 0,01 à 5% en poids d'une base à des températures supérieures à 100°C.